Europäisches Patentamt

European Patent Office

Office européen des brevets

(11) Publication number: **0 293 106**
**A2**

# (12) EUROPEAN PATENT APPLICATION

(21) Application number: **88304262.4**

(22) Date of filing: **11.05.88**

(51) Int. Cl.⁴: **A61B 17/20**

(30) Priority: **26.05.87 NZ 220443**

(43) Date of publication of application:
**30.11.88 Bulletin 88/48**

(84) Designated Contracting States:
**AT BE CH DE ES FR GB GR IT LI LU NL SE**

(71) Applicant: **COOPERS ANIMAL HEALTH NZ LIMITED**
**33 Whakatiki Street**
**Upper Hutt(NZ)**

(72) Inventor: **Harrison, Colin Gibbons**
**32 Kamahi Street**
**Stokes Valley(NZ)**

(74) Representative: **Williams, Trevor John et al**
**J.A. KEMP & CO. 14 South Square Gray's Inn**
**London WC1R 5EU(GB)**

(54) **Scratch vaccinating applicator means.**

(57) The invention comprises vaccine applying apparatus (including applicator head thereof itself) comprising
an applicator head (5) engageable to a reservoir defining means (9), said applicator head (5) having means (3,4,6,11) defining a chamber having a droplet size determining port (2) from which a droplet of liquid from the chambsr can be shaken, an inlet port (11) to be or communicable with the reservoir of said reservoir defining means (9) and means (1) adapted to be used after a droplet has been shaken onto the skin of an animal from the chamber through the droplet size determining port (2) to break the skin, and
means (9) defining a reservoir engageable with said applicator head (5) so as to ensure that liquid therein is communicable with the port (2).

FIG.1

EP 0 293 106 A2

## SCRATCH VACCINATING APPLICATOR MEANS

The present invention relates to improvements in and/or relating to scratch vaccinating applicator means.

Various types of applicator suitable for applying a vaccine which requires a localised disruption to the integrity of the skin after or as a vaccine impregnated liquid, paste or the like is applied thereto are known e.g. see NZ Patent Specification No. 145719. Such means are suitable for certain vaccines but alternate apparatus having certainty of function, low manufacturing cost and/or easy packageability without leakage are also needed.

It is an object of the present invention to provide a scratch vaccinating apparatus which will go at lsast some way to meet the above-mentioned desiderata or which will at least provide the public with a useful choice.

Accordingly in one aspect the present invention consists in vaccine applying apparatus comprising

an applicator head engageable to a reservoir defining means, said applicator head having means defining a chamber having a droplet size determining port from which a droplet of liquid from the chamber can be shaken, an inlet port to be or communicable with the reservoir of said reservoir defining means and means adapted to be used after a droplet has been shaken onto the skin of an animal from the chamber through the droplet size determining port to break the skin, and

means defining a reservoir engageable with said applicator head so as to ensure that liquid therein is communicable with the first mentioned port.

Preferably said reservoir defining means is a bottle or the equivalent container.

Preferably said reservoir defining means includes a screw threaded mouth region capable of screw threadingly receiving the applicator head.

Preferably said applicator head means includes a sealing member e.g. an O-ring or washer.

Preferably said scratching means is in the form of a fixed scratching probe (or needle).

Preferably said probe includes a bifurcated chisel cut scratching end.

Preferably said probe is elongate in nature with the elongate axis thereof aligned with the axis of each of said two ports.

Preferably the size of the inlet port (or ports since there can be more than one if deemed appropriate) is such to ensure that when the apparatus is being shaken to dispense a droplet the surging effect of contents in the reservoir are largely negated during the actual time that the droplet size is being determined by the outlet port.

Preferably the applicator head is in a form substantially as hereinafter described.

In a further aspect of the present invention consists in an applicator head for use in vaccine applying apparatus in accordance with the present invention.

In a still further aspect the present invention consists in applicator head suitable for use in vaccine applying apparatus comprising a first moulded member having an internally threaded skirt engageable with the complementary external thread of an appropriately dimensioned container, an intermediate portion extending axially from the threaded skirt and having means defining a port, and a skirt extending axially from about said port,

a capping member insertable within said skirt so as to define a chamber, said capping member including a droplet size determining port in the cap portion thereof,

and a scratching member secured to or from at least one of the intermediate or threaded skirt regions.

Preferably the applicator head is substantially as hereinafter described.

This invention may also be said broadly to consist in the parts, elements and features referred to or indicated in the specification of the application, individually or collectively, and any or all combinations of any two or more of said parts, elements or features, and where specific integers are mentioned herein which have known equivalents in the art to which this invention relates, such known equivalents are deemed to be incorporated herein as if individually set forth.

The invention consists in the foregoing and also envisages constructions of which the following gives examples.

One preferred form of the present invention will now be described with reference to the accompanying drawings in which

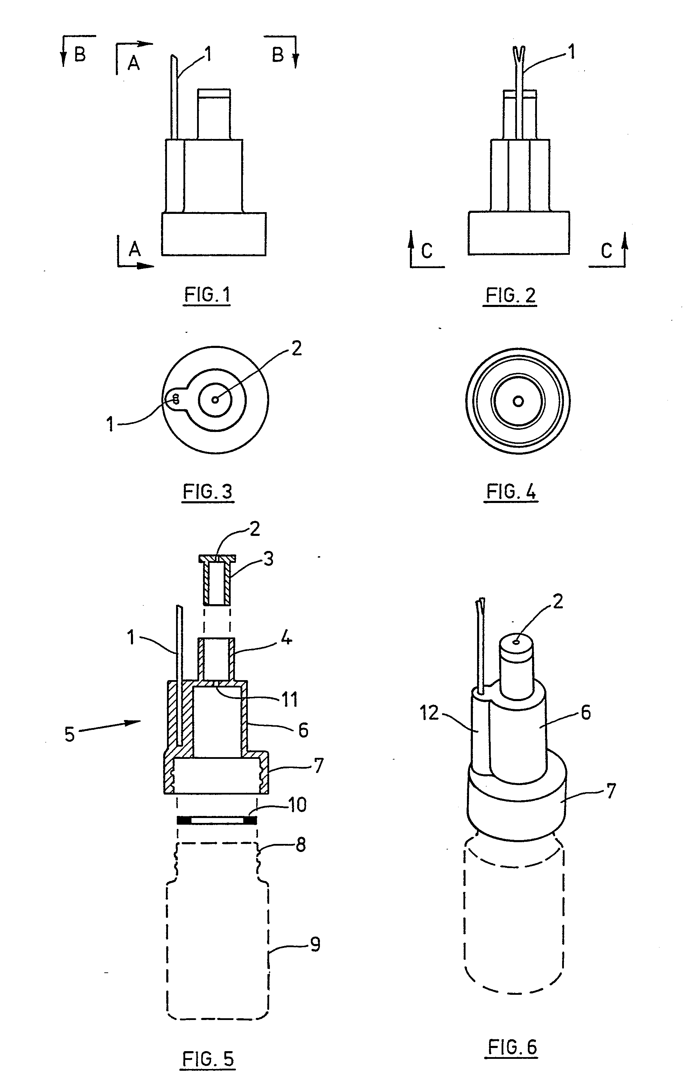

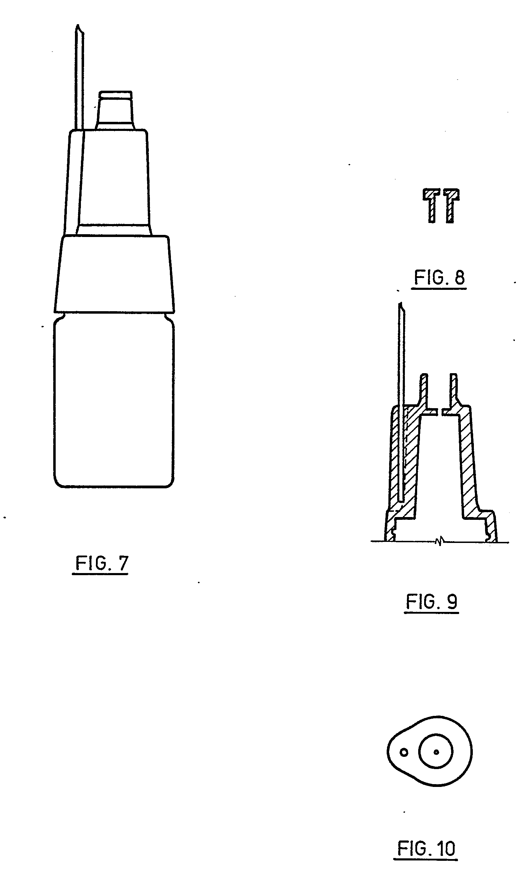

Figure 1 is a side elevational view of a preferred applicator head in accordance with the present invention,

Figure 2 is a view A-A of the head shown in figure one showing the bifurcated end of the probe or scratching needle,

Figure 3 is a view B-B of the applicator head shown in figure 1.

Figure 4 is a view C-C of the mechanism shown in figures 1 and 2,

Figure 5 is an exploded view of apparatus in accordance with the present invention showing in broken outline an externally threaded bottle engageable with the inwardly threaded skirt of the applicator head and also showing the makeup of the applicator head, and

Figure 6 is a perspective view of the arrangement shown in figure 5 when assemble ready for use.

In the preferred form of the present invention the applicator head (preferably of moulded high density polyethylene) includes a bifurcated probe or needle 1 (preferably a steel needle) capable of being used to break the skin of an animal onto which a droplet of vaccine has been shaken from a port 2 in the end of the capping member 3. The capping member 3 preferably of moulded low density polyethylene preferably is simply press fittingly received inwardly of the skirt portion 4. The moulding 5 locates the needle 1 from an intermediate portion 6 ie between the skirt portion 4 and the inwardly threaded skirt 7.

Defined inside the capping member 3 when located in the skirt 4 is a droplet providing chamber defined between parts 2 and 11. For this purpose preferably the port 11 is larger than the port 2 or alternatively there can be a number of such ports. The relative sizing of the ports is critical only to the extent that the droplet size should not be determined by the volume of liquid in the reservoir when the device is shaken i.e. the port 11 should provide some measure of surge resistance to the extent that it affects the droplet size capable of being shaken from opening 2. For example with droplet size determining port being for example from 0.5 to 2.0 mm in diameter an inlet port is best if no more than about 4 times the diameter of that port, preferably about 4 times.

The effect of port size is as follows with, by way of example, the scabby mouth vaccine of Coopers Animal Health NZ Ltd:

| Port Size (mm) mean | Delivery Volume | Standard Deviation |
|---|---|---|
| 0.85 | 0.0115 | 0.0032 |
| 1.00 | 0.03086 | 0.006337 |
| 1.10 | 0.3395 | 0.8977 |

In the preferred form of the present invention the needle 1 is press fittingly engaged, adhesively fixed or moulded into portion 12. The seal 10 is preferably of Nitrile 2.5 SANTOPRENE (trade mark of Monsanto Co).

It is believed that apparatus in accordance with the present invention will find widespread acceptance in the vaccine industry. An appropriate vaccine for inclusion in such a device is that known as the Orf vaccine of Coopers Animal Health NZ Ltd.

## Claims

1. Vaccine applying apparatus comprising an applicator head (5) engageable to a reservoir defining means (9), said applicator head (5) having means (3,4,6,11) defining a chamber having a droplet size determining port (2) from which a droplet of liquid from the chamber can be shaken, an inlet port (11) to be or communicable with the reservoir of said reservoir defining means (9) and means (1) adapted to be used after a droplet has been shaken onto the skin of an animal from the chamber through the droplet size determining port (2) to break the skin, and means (9) defining a reservoir engageable with said applicator head (5) so as to ensure that liquid therein is communicable with the droplet size determining port (2).

2. Apparatus of claim 1 wherein said reservoir defining means (9) is a bottle or the equivalent container.

3. Apparatus of claim 1 or 2 said reservoir defining means (9) includes a screw threaded mouth region (8) capable of screw threadingly receiving the applicator head (5.7).

4. Apparatus of any one of the preceding claims wherein said applicator head means includes an O-ring or washer (10).

5. Apparatus of any one of the preceding claims wherein said scratching means (1) is in the form of a fixed scratching probe or needle (1).

3

6. Apparatus of claim 5 wherein said probe or needle (1) includes a bifurcated chisel cut scratching end.

7. Apparatus of claim 5 or 6 wherein said probe or needle (1) is elongate in nature with the elongate axis thereof aligned with the axis of each of said two ports (2,11).

8. Apparatus of any one of the preceding claims wherein the size of the inlet port (11) (or ports since there can be more than one if deemed appropriate) is such to ensure that when the apparatus is being shaken to dispense a droplet the surging effect of contents in the reservoir are largely negated during the actual time that the droplet size is being determined by the port (2).

9. An applicator head (5) suitable for use in vaccine applying apparatus comprising a first moulded member having an internally threaded skirt (7) engageable with the complementary external thread (8) of an appropriately dimensioned container (9), an intermediate portion (6) extending axially from the threaded skirt (7) and having means defining a port (11), and a skirt (4) extending axially from about said port (11),

a capping member (3) insertable within said skirt (4) so as to define a chamber, said capping member (3) including a droplet size determining port (2) in the cap portion thereof,

and a scratching member (1) secured to or from at least one of the intermediate or threaded skirt regions (6,7).

10. Apparatus of any one of claims 1 to 8 wherein the applicator head is as claimed in claim 9.

FIG. 1

FIG. 2

FIG. 3

FIG. 4

FIG. 5

FIG. 6

FIG. 7

FIG. 8

FIG. 9

FIG. 10